# EUROPEAN PATENT APPLICATION

(11) **EP 2 186 784 A2**
(43) Date of publication of application: **19.05.2010**
(21) Application number: 10151833.0
(22) Date of filing: 27.01.2010
(51) Int. Cl.: C07C 5/333, C07C 7/13, C07C 11/02, C07C 303/06, C07C 309/20

(54) **Process for the preparation and recovery of olefins**

(71) Applicant: Shell Internationale Research Maatschappij B.V., 2596 HR The Hague (NL)
(72) Inventor: Dirkzwager, Hendrik, 1031 HW, Amsterdam (NL); Moene, Robert, 1031 HW, Amsterdam (NL)

(57) **Abstract**

A process for the preparation and recovery of olefins, said process comprising:-
(i) passing a feed stream comprising paraffins having in the range of from 20 to 28 carbon atoms to a dehydrogenation zone, operating the dehydrogenation zone at dehydrogenation conditions sufficient to dehydrogenate paraffins, and recovering from the dehydrogenation zone a dehydrogenation product stream comprising monoolefins and paraffins wherein the monoolefins in the dehydrogenated product stream have in the range of from 20 to 28 carbon atoms; and
(ii) passing the dehydrogenation product stream to a separation zone and contacting said dehydrogenation product stream with a bed of adsorbent under conditions which cause the selective retention of the monoolefins having in the range of from 20 to 28 carbon atoms on the adsorbent and recovering the retained monoolefins having in the range of from 20 to 28 carbon atoms from the adsorbent by contacting the adsorbent with a desorbent.

## Description

### Field of the Invention

The present invention relates to a process for the preparation and recovery of olefins, in particular olefins having in the range of from 20 to 28 carbon atoms, from paraffins.

### Background of the Invention,

Heavy internal olefins (e.g. C15-C30) are needed for the production of Internal Olefin Sulphonates (IOS) and/or their derivatives (according to EP 351928 A1).

Branched and linear heavy internal olefins are useful surfactant-precursors to be applied in enhanced oil recovery (EOR).

Furthermore, the internal olefin derived alcohols and/or alkoxylates may serve this purpose after conversion to anionic surfactants by sulphation.

Appropriate internal higher olefin feeds can be obtained from the Shell Higher Olefin Process (SHOP) process and 1-olefin production processes as employed by Chevron-Phillips Chemical Company or INEOS (BP-AMOCO). However, as it is expected that large amounts of surfactants and hence, heavy internal olefins (e.g. C15-C30), are required for EOR, it is desirable if such olefins can also be obtained from other sources.

The dehydrogenation of higher paraffins leads to a mixture of olefins which mostly contain internal double bonds. As described in Encyclopedia of Chemical Processing and Design, 1982, Vol. 15, pp 266-284, a commercial process for the dehydrogenation of paraffins according is UOP's "PACOL" process. This process gives rise to the production of paraffin-diluted internal olefins (generally < 20 percent by weight (wt. %) olefin content), as at higher conversions per pass dienes and aromatics are formed as by-products to a large extent and moreover, coking phenomena are encountered in the "PACOL" dehydrogenation unit. However, said process has been directed to preparing lower carbon number olefins, i.e. olefins having in the range of from 10 to 13 carbon atoms, for use, in particular, in the manufacture of alkyl benzenes as feedstock for detergent manufacture.

For example, US 7102044 B1 describes a process for the removal of oxygenates from a paraffin-rich or olefin-rich paraffin stream which comprises passing a feed stream, comprising one or more C₁₀ to C₁₅ feed paraffins or C₁₀ to C₁₅ olefin-rich paraffin stream and one or more oxygenates through an adsorbent bed comprising one or more adsorbents selected from silica gel, activated alumina and sodium X zeolites to remove essentially all of said oxygenates; and recovering said paraffins. Whilst the process of US 7102044 B1 may comprise an optional dehydrogenation step on the C₁₀ to C₁₅ feed paraffins after the afore-mentioned adsorptive step, it should be noted that US 7102044 B1 is in no way directed to the production of higher carbon number olefins for use in the manufacture of surfactants for enhanced oil recovery applications.

US 2007/0203387 A1 describes a continuous process for removing aromatic by-products from a feedstock containing aliphatic mono-olefins having from 6 to 22 carbon atoms per molecule and aromatic by-products having from 7 to 22 carbon atoms per molecule.

Whilst another embodiment of US 2007/0203387 A1 describes an integrated paraffin dehydrogenation and benzene alkylation process on a feedstock comprising a paraffin having from 6 to 22 carbon atoms per molecule, it is of note that the teaching and examples of US 2007/0203387 A1 are directed to paraffins having much lower carbon numbers of up to and including 13 carbon atoms. The Examples in US 2007/0203387 A1 are prophetic and there are no actual working examples therein.

In summary, US 2007/0203387 A1 contains no teaching therein which is concerned with the production of higher carbon number olefins for use in the manufacture of surfactants for enhanced oil recovery applications.

Hence, a cheap and straightforward technique is required for preparing and recovering heavy olefins, and in particular heavy internal olefins, for use as surfactant precursors.

### Summary of the Invention

According to the present invention there is provided a process for the preparation and recovery of olefins, said process comprising:-
(i) passing a feed stream comprising paraffins having in the range of from 20 to 28 carbon atoms to a dehydrogenation zone, operating the dehydrogenation zone at dehydrogenation conditions sufficient to dehydrogenate paraffins, and recovering from the dehydrogenation zone a dehydrogenation product stream comprising monoolefins and paraffins wherein the monoolefins in the dehydrogenated product stream have in the range of from 20 to 28 carbon atoms; and
(ii) passing the dehydrogenation product stream to a separation zone and contacting said dehydrogenation product stream with a bed of adsorbent under conditions which cause the selective retention of the monoolefins having in the range of from 20 to 28 carbon atoms on the adsorbent and recovering the retained monoolefins having in the range of from 20 to 28 carbon atoms from the adsorbent by contacting the adsorbent with a desorbent.

### Detailed Description of the Invention

The feed stream in the process of the present invention may be any stream comprising paraffins having in the range of from 20 to 28 carbon atoms.

However, in a preferred embodiment, said feed stream is a Fischer-Tropsch derived feed stream, that is to say, a product stream from Fischer-Tropsch synthesis (e.g. a gas-to-liquids (GTL), a biomass-to-liquids (BTL) or a coal-to-liquids (CTL) product).

The Fischer-Tropsch process is well known to those skilled in the art and can be used for the conversion of synthesis gas (from hydrocarbonaceous feed stocks) into liquid and/or solid hydrocarbons. Generally, the feed stock (e.g. natural gas, associated gas and/or coal-bed methane, heavy and/or residual oil fractions, coal, biomass) is converted in a first step into a mixture of hydrogen and carbon monoxide (this mixture is often referred to as "synthesis gas" or "syngas").

The synthesis gas is then fed into a reactor where it is converted in one or more steps over a suitable catalyst at elevated temperature and pressure into mainly paraffinic compounds and water. The obtained paraffinic compounds range from methane to high molecular weight molecules. The obtained high molecular weight molecules can comprise up to 200 carbon atoms, or, under particular circumstances, even more carbon atoms.

Numerous types of reactor systems have been developed for carrying out the Fischer-Tropsch reaction. For example, Fischer-Tropsch reactor systems include fixed bed reactors, especially multi-tubular fixed bed reactors, fluidised bed reactors, such as entrained fluidised bed reactors and fixed fluidised bed reactors, and slurry bed reactors such as three-phase slurry bubble columns and ebulated bed reactors.

Catalysts used in the Fischer-Tropsch synthesis often comprise a carrier based support material and one or more metals from Group VIII (IUPAC 8-10) of the Periodic Table of Elements, especially from the cobalt or iron groups, optionally in combination with one or more metal oxides and/or metals as promoters selected from zirconium, titanium, chromium, vanadium and manganese, especially manganese. Such catalysts are known in the art and have been described for example, in the specifications of WO 97/00231 A and US 4595703 A.

The synthesis gas can be provided by any suitable means, process or arrangement. This includes partial oxidation and/or reforming of a hydrocarbonaceous feedstock as is known in the art. To adjust the H₂/CO ratio in the synthesis gas, carbon dioxide and/or steam may be introduced into the partial oxidation process. The H₂/CO ratio of the synthesis gas is suitably between 1.5 and 2.3, preferably between 1.6 and 2.0.

The synthesis gas comprising predominantly hydrogen, carbon monoxide and optionally nitrogen, carbon dioxide and/or steam is contacted with a suitable catalyst in the catalytic conversion stage, in which the hydrocarbons are formed. Suitably at least 70 v/v% of the synthesis gas is contacted with the catalyst, preferably at least 80 %, more preferably at least 90 %, still more preferably all the synthesis gas.

A steady state catalytic hydrocarbon synthesis process may be performed under conventional synthesis conditions known in the art. Typically, the catalytic conversion may be effected at a temperature in the range of from 100 to 600 °C, preferably from 150 to 350 °C, more preferably from 175 to 275 °C, most preferably 200 to 260 °C. Typical total pressures for the catalytic conversion process are in the range of from 5 to 150 bar absolute, more preferably from 5 to 80 bar absolute. In the catalytic conversion process mainly C₅+ hydrocarbons are formed.

A suitable regime for carrying out the Fischer-Tropsch process with a catalyst comprising particles with a size of least 1 mm is a fixed bed regime, especially a trickle flow regime. A very suitable reactor is a multi-tubular fixed bed reactor.

Products of the Fischer-Tropsch synthesis may range from methane to heavy paraffin waxes. Preferably, the production of methane is minimised and a substantial portion of the hydrocarbons produced have a carbon chain length of a least 5 carbon atoms. Preferably, the amount of C₅+ hydrocarbons is at least 60 wt. % of the total product, more preferably, at least 70 wt. %, even more preferably, at least 80 wt. %, most preferably at least 85 wt. %.

The hydrocarbons produced in the Fischer-Tropsch process are suitably C3-200 hydrocarbons, more suitably C4-150 hydrocarbons, especially C5-100 hydrocarbons, or mixtures thereof. These hydrocarbons or mixtures thereof are liquid or solid at temperatures between 5 and 30 °C (1 bar), especially at about 20 °C (1 bar), and usually are paraffinic in nature, while up to 30 wt. %, preferably up to 15 wt. %, of either olefins or oxygenated compounds may be present.

Depending on the catalyst and the process conditions used in a Fischer-Tropsch reaction, various proportions of normally gaseous hydrocarbons, normally liquid hydrocarbons and optionally normally solid hydrocarbons are obtained. It is often preferred to obtain a large fraction of normally solid hydrocarbons. These solid hydrocarbons may be obtained up to 90 wt. % based on total hydrocarbons, usually between 50 and 80 wt. %.

A part may boil above the boiling point range of the so-called middle distillates. The term "middle distillates", as used herein, is a reference to hydrocarbon mixtures of which the boiling point range corresponds substantially to that of kerosene and gasoil fractions obtained in a conventional atmospheric distillation of crude mineral oil. The boiling point range of middle distillates generally lies within the range of from 150 to 360 °C.

The higher boiling range paraffinic hydrocarbons, if present, may be isolated and subjected to a catalytic hydrocracking step, which is known per se in the art, to yield the desired middle distillates. The catalytic hydrocracking is carried out by contacting the paraffinic hydrocarbons at elevated temperature and pressure and in the presence of hydrogen with a catalyst containing one or more metals having hydrogenation activity, and supported on a support comprising an acidic function. Suitable hydrocracking catalysts include catalysts comprising metals selected from Groups VIB (IUPAC 6) and VIII (IUPAC 8-10) of the Periodic Table of Elements. Preferably, the hydrocracking catalysts contain one or more noble metals from Group VIII (IUPAC 8-10) of the Periodic Table of Elements. Preferred noble metals are platinum, palladium, rhodium, ruthenium, iridium and osmium. Most preferred catalysts for use in the hydrocracking stage are those comprising platinum.

The amount of catalytically active noble metal present in the hydrocracking catalyst may vary within wide limits and is typically in the range of from 0.05 to 5 parts by weight per 100 parts by weight of the support material. The amount of non-noble metal present is preferably 5-60 %, more preferably 10-50 %.

Suitable conditions for the catalytic hydrocracking are known in the art. Typically, the hydrocracking is effected at a temperature in the range of from 175 to 400 °C. Typical hydrogen partial pressures applied in the hydrocracking process are in the range of from 10 to 250 bar.

The product of the hydrocarbon synthesis and consequent hydrocracking suitably comprises mainly normally liquid hydrocarbons, beside water and normally gaseous hydrocarbons. By selecting the catalyst and the process conditions in such a way that especially normally liquid hydrocarbons are obtained, the product obtained ("syncrude") may be transported in the liquid form or be mixed with any stream of crude oil without creating any problems as to solidification and or crystallization of the mixture. It is observed in this respect that the production of heavy hydrocarbons, comprising large amounts of solid wax, are less suitable for mixing with crude oil while transport in the liquid form has to be done at elevated temperatures, which is less desired.

Thus, a Fischer-Tropsch derived feed stream for use in the process of the present invention may have undergone the steps of hydroprocessing, hydrogenation, hydroisomerisation and/or hydrocracking. Said feed stream may also be a fuel, preferably gasoil, a waxy raffinate or a base oil.

Consequently, said Fischer-Tropsch derived feed stream may be any product stream from a Fischer-Tropsch process which comprises paraffins having in the range of from 20 to 28 carbon atoms. That is to say, the Fischer-Tropsch derived feed stream may be the direct product stream from the Fischer-Tropsch paraffin synthesis unit (HPS) or it may be a product stream derived therefrom after subsequent processing such as hydrogenation, for example as described below.

In the Fischer-Tropsch process, light ends (C5-) and a Light Detergent Feedstock (LDF, C10-C13) are typically removed by distillation from the product stream from the HPS unit. The Light Detergent Feedstock (LDF, C10-C13) stream is subsequently subjected to hydrogenation. The remainder of the HPS effluent is then typically sent to a hydrocracking unit (HPC). The effluent from the HPC unit is then typically distilled in a syncrude distillation unit (SCD) to produce products ranging from LPG to Gasoil. The heavy ends from the SCD unit are sent to vacuum distillation unit (HVU) where fractions to be used for base oils are removed before the heavies are recycled back to the HPC unit. The base oil fractions are then sent to a catalytic dewaxing unit (CDW) before being distilled into final base oil product fractions.

Hence, additional sources of paraffins having in the range of from 20 to 28 carbon atoms within the Fischer-Tropsch process not only include product streams directly from the HPS unit, but may also include product streams from the hydrocracking unit (HPC) and certain base oil product fractions.

A Fischer-Tropsch derived feed stream for use in the process of the present invention may comprise mixtures of linear and/or branched olefins and paraffins. Oil reservoirs have variable characteristics such as crude oil type, salinity and temperature. Consequently, surfactants such as internal olefin sulphonates for use in enhanced oil recovery applications are tailored dependent upon the specific characteristics of the oil reservoir being treated.

Accordingly, in one preferred embodiment of the present invention the feed stream comprises paraffins having in the range of from 20 to 24 carbon atoms, whilst in another preferred embodiment of the present invention the feed stream comprises paraffins having in the range of from 24 to 28 carbon atoms. In particularly preferred embodiments, said feed streams are derived from a Fischer-Tropsch process.

Depending upon from wherein the feed stream is derived in the Fischer-Tropsch process, the feed stream for use in the process of the present invention may comprise different levels of paraffins, in particular branched paraffins.

For example, in one embodiment of the present invention, when the Fischer-Tropsch derived feed stream is from the HPS unit, it is preferred that paraffins having in the range of from 20 to 28 carbon atoms, more preferably in the range of from 20 to 24 carbon atoms or 24 to 28 carbon atoms, are present in the feed stream in an amount of at least 5 wt. %, with respect to the total weight of said Fischer-Tropsch derived feed stream and that said feed stream comprises at least 5 wt. % of branched paraffins having in the range of from 20 to 28 carbon atoms, more preferably in the range of from 20 to 24 carbon atoms or 24 to 28 carbon atoms, with respect to the total weight of paraffins in said carbon number range in the Fischer-Tropsch derived feed stream.

In another embodiment of the present invention, when the Fischer-Tropsch derived feed stream is from the HPC unit, it is preferred that paraffins having in the range of from 20 to 28 carbon atoms, more preferably in the range of from 20 to 24 carbon atoms or 24 to 28 carbon atoms, are present in the feed stream in an amount of at least 15 wt. %, with respect to the total weight of said Fischer-Tropsch derived feed stream and that said feed stream comprises at least 50 wt. % of branched paraffins having in the range of from 20 to 28 carbon atoms, more preferably in the range of from 20 to 24 carbon atoms or 24 to 28 carbon atoms, with respect to the total weight of paraffins in said carbon number range in the Fischer-Tropsch derived feed stream.

In a further embodiment of the present invention, when the Fischer-Tropsch derived feed stream is a base oil product fraction, it is preferred that paraffins having in the range of from 20 to 28 carbon atoms, more preferably in the range of from 20 to 24 carbon atoms or 24 to 28 carbon atoms, are present in the feed stream in an amount of at least 50 wt. %, with respect to the total weight of said Fischer-Tropsch derived feed stream and that said feed stream comprises at least 90 wt. % of branched paraffins having in the range of from 20 to 28 carbon atoms, more preferably in the range of from 20 to 24 carbon atoms or 24 to 28 carbon atoms, with respect to the total weight of paraffins in said carbon number range in the Fischer-Tropsch derived feed stream.

The dehydrogenation catalyst used in the dehydrogenation zone of the process of the present invention may be in a fixed bed, a moving catalyst bed or a fluidised bed.

It will be appreciated that the dehydrogenation zone may actually comprise one or more catalyst-containing dehydrogenation reaction zones with heat exchangers there between to ensure that the desired reaction temperature is maintained at the entrance to each reaction zone. One or more hot hydrogen-rich gas streams may be introduced between a first and a second dehydrogenation reaction zone to increase the temperature of a stream passing from the first to the second reaction zone, as disclosed in US 5491275 A and US 5689029 A.

The or each dehydrogenation reaction zone may be operated in a continuous-type or batch-type manner. Each dehydrogenation reaction zone may contain one or more catalyst beds. Paraffins may contact any catalyst bed in an upward-, downward-, or radial-flow fashion. In a particularly compact and efficient arrangement, the contacting of the catalyst with paraffins and heat exchanging may be accomplished in a heat exchanging reactor. One example of such a reactor is an isothermal reactor design using interleaved layers of plate heat exchange elements, which is described in US 5405586 A.

Another example of a reactor arrangement is disclosed in US 5525311 A, where a reactant stream indirectly contacts a heat exchange stream and where an arrangement of corrugated heat exchange plates is used to control temperature conditions by varying the number and/or the arrangement of the corrugations along the plates.

The choice of a dehydrogenation catalyst is not limited in the process of the present invention.

Dehydrogenation catalysts are well known in the prior art. For example, suitable procedures for preparing catalysts and performing dehydrogenation include US 3274287 A, US 3315007 A, US 3315008 A, US 3745112 A, US 4048246 A, US 4079097 A, US 4080394 A, US 4304950 A, US 4430517 A, US 4458098 A, US 4506032 A, US 4595673 A, US 4677237 A, US 4716143 A, US 4762960 A, US 4786625 A, US 4827072 A, US 5012021 A, US 6187981 B1, US 6756515 B1, US 2003/0202934 A1, CA 928330 A, WO 02/41990 A1, WO 2004/072004 and WO 2005/037753.

Preferred dehydrogenation catalyst compositions for use in the process of the present invention may comprise at least one platinum group (Group VIII (IUPAC 8-10) of the Periodic Table of Elements) metal, a promoter metal, a modifier metal, and a porous carrier material.

The platinum group (Group VIII (IUPAC 8-10) of the Periodic Table of Elements) metals which may be used include platinum, palladium, rhodium, iridium, ruthenium, and osmium.

In certain embodiments, the platinum group (Group VIII (IUPAC 8-10) of the Periodic Table of Elements) metal may be dispersed throughout the dehydrogenation catalyst composition.

The platinum group (Group VIII (IUPAC 8-10) of the Periodic Table of Elements) metal may be incorporated into the catalyst composition by techniques known in the art (e.g., co-precipitation, co-gelation, ion exchange, impregnation, deposition from a vapour phase or from an atomic source) before incorporation of other catalytic components. In some embodiments, the platinum group (Group VIII (IUPAC 8-10) of the Periodic Table of Elements) metal may be incorporated into the catalyst composition during incorporation of other catalytic components. In other embodiments, the platinum group (Group VIII (IUPAC 8-10) of the Periodic Table of Elements) metal may be incorporated into the catalyst composition after incorporation of other catalytic components. In certain embodiments, the platinum group (Group VIII (IUPAC 8-10) of the Periodic Table of Elements) metal may be incorporated by impregnation of' the porous carrier material with a solution or suspension of a decomposable compound of the platinum group (Group VIII (IUPAC 8-10) of the Periodic Table of Elements) metal. For example, platinum may be added to a catalytic support by commingling the platinum with an aqueous solution of chloroplatinic acid. In other embodiments, optional components (e.g., nitric acid) may be added to the impregnating solution to assist in dispersing or fixing the platinum group (Group VIII (IUPAC 8-10) of the Periodic Table of Elements) metal in the final catalyst composition.

Promoter metals may be conveniently selected from the group consisting of tin, germanium, rhenium, gallium, bismuth, lead, indium, cerium, zinc, and mixtures thereof.

In some embodiments, the promoter metal may exist within the catalyst composition in an oxidation state above that of the platinum group (Group VIII (IUPAC 8-10) of the Periodic Table of Elements) metal. The promoter metal may be present as an oxide. In certain embodiments, a promoter metal may be combined with a carrier material. In some embodiments, the promoter metal may be combined with the other catalyst components. In other embodiments, the promoter metal may be dispersed throughout the catalyst composition.

The promoter metal may be incorporated in the catalyst composition according to generally known methods (e.g., co-precipitation, co-gelation, ion exchange and impregnation) before other catalyst components are incorporated. In some embodiments, the promoter metal may be incorporated during incorporation of other catalyst components. In other embodiments, the promoter metal may be incorporated after other catalyst components are incorporated. In some embodiments, for example, a tin component may be incorporated by co-gelation with the porous carrier material. The tin may be incorporated in an alumina carrier material by mixing a soluble tin compound (e.g., stannous or stannic chloride) with an alumina hydrosol. A gelling agent (e.g., hexamethylenetetraamine) may be added to the tin-alumina hydrosol mixture. The tin-alumina hydrosol mixture may be dropped into an oil bath to form spheres containing alumina and tin. In other embodiments, a germanium component may be impregnated into a carrier material with a solution or suspension of a decomposable compound of germanium (e.g., germanium tetrachloride dissolved in an alcohol). In other embodiments, a lead component may be impregnated from a solution of lead nitrate in water.

Modifier metals may conveniently be selected from the group consisting of alkali metals, alkaline earth metals and mixtures thereof.

The alkali and alkaline earth metals which can be used as modifier metals in the dehydrogenation catalyst composition include lithium, sodium, potassium, caesium, rubidium, beryllium, magnesium, calcium, strontium, and barium. Preferred modifier metals are lithium, potassium, sodium, and caesium with lithium and potassium being especially preferred. The modifier metal may exist in the final catalyst composition in an oxidation state above that of the platinum group (Group VIII (IUPAC 8-10) of the Periodic Table of Elements) metal. The modifier metal may be present as an oxide. In some embodiments, the modifier metal may be combined with the porous carrier material. In certain embodiments, the modifier metal may be combined with other dehydrogenation catalyst components.

In other embodiments, the modifier metal may be dispersed throughout the catalytic composite. An amount of the modifier metal may range from 0.01 to 15 wt. %, calculated on an elemental basis, of the final catalyst composition. In some embodiments, the dehydrogenation catalyst composition may include in the range of from 1 to 3 wt. % of potassium. In certain embodiments, an atomic ratio of the modifier metal to the platinum group (Group VIII (IUPAC 8-10) of the Periodic Table of Elements) metal may be greater than at least 10.

The modifier metal may be incorporated in the catalyst composition according to generally known methods (e.g., co-precipitation, co-gelation, ion exchange or impregnation) before other catalyst components are incorporated. In some embodiments, the modifier metal may be incorporated during incorporation of other catalyst components. In other embodiments, the modifier metal may be incorporated after other catalyst components are incorporated. For example, a potassium component may be impregnated into the porous carrier material with a solution of potassium nitrate. An atomic ratio of alkali or alkaline earth component to platinum group (Group VIII (IUPAC 8-10) of the Periodic Table of Elements) metal may be at least 10. In certain embodiments, an atomic ratio of the alkali or alkaline earth component to the platinum group (Group VIII (IUPAC 8-10) of the Periodic Table of Elements) metal may range from 15 to 25.

The porous carrier material used in the dehydrogenation catalyst composition may include a porous, absorptive support with high surface area in the range of from 25 m²/g to 500 m²/g. The porous carrier material may have a melting point greater than the conditions utilised in the dehydrogenation zone.

Examples of carrier materials include, but are not limited to, activated carbon, coke, charcoal, silica, silica gel, silicon carbide, synthetically prepared and/or naturally occurring clays and silicates, refractory inorganic oxides, crystalline zeolitic aluminosilicates, naturally occurring or synthetically prepared mordenite and/or faujasite, spinels or combinations of thereof.

In some embodiments, clays and silicates may or may not be acid treated (e.g., attapulgite, china clay, diatomaceous earth, fuller's earth, kaolin, kieselguhr, ceramics, porcelain, crushed firebrick, bauxite).

Examples of refractory inorganic oxides include alumina, titanium dioxide, zirconium dioxide, chromium oxide, beryllium oxide, vanadium oxide, cerium oxide, hafnium oxide, zinc oxide, magnesia, boria, thoria, silica-alumina, silica-magnesia, chromia-alumina, alumina-boria, and silica-zirconia.

Zeolitic aluminosilicates may be, in some embodiments, in the hydrogen form. In other embodiments, zeolitic aluminosilicates may be in a form that may be exchanged with metal cations. Examples of spinels include, but are not limited to, MgAl₂O₄, FeAl₂O₄, ZnAl₂O₄, CaAl₂O₄, and other like compounds having the formula MO-Al₂O₃ in which M is a metal having a valence of 2.

In certain embodiments, an alumina carrier material used in a dehydrogenation catalyst may be prepared in any suitable manner from synthetic or naturally occurring raw materials. The alumina carrier may be formed in any desired shape (e.g., spheres, pills, cakes, extrudates, powders, granules). The alumina carrier may be utilized in any particle size. In certain embodiments, a sphere shape may be utilized. The particle may be about 1/16 inch in diameter. In certain embodiments, a particle diameter of less than about 1/32 inch may be utilized.

Alumina spheres may be prepared, in some embodiments, by converting aluminium metal into an alumina sol. An alumina sol may be prepared by reacting aluminium metal with a suitable peptizing acid and water. The resulting alumina sol and a gelling agent may be dropped into an oil bath to form spherical particles of an alumina gel. The resulting alumina gel may be converted to gamma- or eta-alumina carrier material using known techniques (e.g., by aging, drying and calcining).

In other embodiments, alumina cylinders may be prepared by mulling alumina powder with water and a suitable peptizing agent (e.g., nitric acid) to form an extrudable composition. The composition may be extruded through a suitably sized die then cut to form extrudate particles. Other shapes of the alumina carrier material may be prepared by conventional methods. After the alumina particles are shaped, they may be dried and calcined. The alumina carrier may be subjected to intermediate treatments (e.g., washing with water or a solution of ammonium hydroxide) during preparation.

Preferred porous carrier materials are refractory inorganic oxides selected from alpha alumina, theta alumina, cordierite, zirconia, titania, and mixtures thereof. Particularly preferred porous carrier materials are alpha alumina and cordierite.

Although the concentration of each metal component can vary substantially, it is preferable that the platinum group (Group VIII (IUPAC 8-10) of the Periodic Table of Elements) metal be present in a concentration in the range of from 0.01 to 5 wt. % on an elemental basis of the total weight of the catalyst composition, more preferably in the range of from 0.1 to 1.0 wt. % and most preferably in the range of from 0.05 to 1.0 wt. % on an elemental basis of the total weight of the catalyst composition.

The promoter metal is preferably present in an amount in the range of from 0.01 to 5 wt. %, more preferably in the range of from 0.05 to 5 wt. % of the total weight of the catalyst composition.

The modifier metal is preferably present in an amount in the range of from 0.1 to 5 wt. % and more preferably in the range of from 2 to 4 wt. % of the total weight of the catalyst composition.

The atomic ratio of the platinum group (Group VIII (IUPAC 8-10) of the Periodic Table of Elements) metal to modifier metal may vary in the range of from 0.05 to 5. In particular, when the modifier metal is tin, the atomic ratio is typically in the range of from 0.1:1 to 5:1 and preferably in the range of from 0.5:1 to 3:1. When the modifier metal is germanium the ratio is typically in the range of from 0.25:1 to 5:1 and when the promoter metal is rhenium, the ratio is typically in the range of from 0.05:1 to 2.75:1.

In some embodiments, the dehydrogenation catalyst composition may additionally comprise a halogen component. The halogen component may include, but is not limited to, fluorine, chlorine, bromine, iodine or mixtures thereof. The halogen component may be present in a combined state with the porous carrier material. In certain embodiments, a halogen component may be dispersed throughout the catalyst composition. A halogen component may range from 0.2 to 15 wt. %, calculated on an elemental basis, of the final catalyst composition. In certain embodiments, the dehydrogenation catalyst composition may contain in the range of from 1 to 3 wt. % chlorine.

In certain embodiments, the catalyst composition may include at least 0.2 wt. %, calculated on an elemental basis, of a halogen component. The presence of a halogen component in the catalyst composition may improve the dehydrogenation activity of the catalyst. In some embodiments, the presence of an active halogen component may suppress carbon formation on the catalyst composition during the dehydrogenation process. An additional advantage of the catalyst composition may be that undesirable isomerisation or cracking side reactions may be inhibited. In certain embodiments, the halogen content may increase the acidity of the catalyst composition. Consequently, the acidity may be lowered by steaming the dehydrogenation catalyst composition to remove excess halogen therefrom.

A halogen component may be incorporated in the catalyst composition in any suitable manner. The incorporation of the halogen may be before preparation of the porous carrier material. In some embodiments, incorporation of a halogen may be during incorporation of other catalyst components. In other embodiments, incorporation of a halogen may be after other catalyst components are incorporated. In certain embodiments, an alumina sol carrier may contain a halogen, which may contribute to at least some portion of the halogen content in the final catalyst composition. In some embodiments, a halogen component, or a portion thereof, may be added to the catalyst composition during the incorporation of the carrier material with other catalyst components (e.g., using chloroplatinic acid to impregnate the platinum component). In other embodiments, a halogen component or a portion thereof may be added to the catalyst composition by contacting the catalyst with the halogen. In some embodiments, a halogen may be added to the catalyst as a compound, solution, suspension or dispersion containing the halogen, (e.g., hydrochloric acid) before or after other catalyst components are incorporated with the carrier material. In some embodiments, a halogen component may be added to the catalyst composition by adding the halogen or a compound, solution, suspension or dispersion containing the halogen (e.g., propylene dichloride) to the feed stream. In other embodiments, a halogen component may be added to the recycle gas during operation of the dehydrogenation unit.

In some embodiments, the dehydrogenation catalyst composition may include a sulphur component in an amount ranging from 0.01 to 10 wt. %, calculated on an elemental basis, of the final catalyst composition.

The sulphur component may be incorporated into the catalyst composition in any suitable manner. In certain embodiments, sulphur or a compound containing sulphur (e.g., hydrogen sulphide or a lower molecular weight mercaptan) may be contacted with the catalyst composition in the presence of hydrogen at a temperature ranging from about 10 °C to about 540 °C under anhydrous conditions. A hydrogen to sulphur ratio, in some embodiments, may be about 100.

The dehydrogenation catalyst composition, in some embodiments, may also contain other, additional components or mixtures thereof, which act alone or in concert, as catalyst modifiers to further improve catalyst activity, selectivity or stability.

Examples of catalyst modifiers include, but are not limited to, antimony, arsenic, beryllium, bismuth, cadmium, calcium, chromium, cobalt, copper, gallium, gold, indium, iron, lithium, manganese, molybdenum, nickel, rhenium, scandium, silver, tantalum, thallium, titanium, tungsten, uranium, zinc and zirconium. Catalyst modifiers may be added in any suitable manner to the porous carrier material during preparation of the dehydrogenation catalyst composition. In other embodiments, catalyst modifiers may be added in any suitable manner after preparation of the dehydrogenation catalyst composition. In some embodiments, catalyst modifiers may be added in any suitable manner to the catalyst composition before other catalyst components are incorporated. In certain embodiments, catalyst modifiers may be added during incorporation of other catalyst components. In other embodiments, catalyst modifiers may be added after other catalyst components are incorporated.

A description of a preferred dehydrogenation catalyst may be found in US 4506032 A.

In a particularly preferred embodiment, the dehydrogenation catalyst is a layered composition as described in US 6187981 B1. Said layered composition comprises an inner core and an outer layer bonded to the inner core, where the outer layer comprises a refractory inorganic oxide having uniformly dispersed thereon at least one platinum group (Group VIII (IUPAC 8-10) of the Periodic Table of Elements) metal and at least one promoter metal, and where at least one modifier metal is dispersed on the catalyst composition. Preferably, the outer layer is bonded to the inner core to the extent that the attrition loss is less than 10 wt. % based on the weight of the outer layer.

As described in US 6187981 B1, said preferred catalyst composition comprises an inner core composed of a material which has substantially lower adsorptive capacity for catalytic metal precursors, relative to the outer layer. Some of the inner core materials are also not substantially penetrated by liquids, e.g., metals. Examples of the inner core material include, but are not limited to, refractory inorganic oxides, silicon carbide, and metals. The materials which form the inner core of the catalyst composition may be formed into a variety of shapes such as pellets, extrudates, spheres, or irregularly shaped particles, although not all materials can be formed into each shape. Preparation of the inner core can be done by means known in the art such as oil dropping, pressure moulding, metal forming, pelletising, granulation, extrusion, rolling methods, and marumerising. A spherical inner core is preferred. The inner core whether spherical or not has an effective diameter in the range of from 0.05 mm (0.0020 in) to 5 mm (0.2 in), preferably in the range of from 0.8 mm (0.031 in) to 3 mm (0.12 in). For a non-spherical inner core, the effective diameter is defined as the diameter the shaped article would have if it were moulded into a sphere. Once the inner core is prepared, it is calcined at a temperature in the range of from 400 °C (752 °F) to 1800 °C (3272 °F). When the inner core comprises cordierite, it is calcined at a temperature in the range of from 1000 °C (1832 °F) to 1800 °C (3272 °F).

The inner core of the catalyst composition is coated with a layer of a refractory inorganic oxide which is different from the inorganic oxide which may be used as the inner core and will be referred to herein as the outer refractory inorganic oxide. This outer refractory inorganic oxide is one which has good porosity, has a surface area of at least 20 m²/g, and preferably at least 50 m²/g, has an apparent bulk density of from about 0.2 g/ml to about 1.0 g/ml, and is chosen from the group consisting of gamma alumina, delta alumina, eta alumina, and theta alumina. Preferred outer refractory inorganic oxides are gamma alumina and eta alumina.

US 2620314 A describes a method of preparing a gamma alumina by the oil drop method. Said method comprises forming an aluminium hydrosol by prior art techniques and preferably by reacting aluminium metal with hydrochloric acid; combining the hydrosol with a suitable gelling agent, e.g., hexamethylenetetraamine; and dropping the resultant mixture into an oil bath maintained at elevated temperatures (about 93 °C (199 °F)). The droplets of the mixture remain in the oil bath until they set and form hydrogel spheres. The spheres are then continuously withdrawn from the oil bath and typically subjected to specific aging and drying treatments in oil and ammoniacal solutions to further improve their physical characteristics. The resulting aged and gelled spheres are then washed and dried at a relatively low temperature in the range of from 80 °C (176 °F) to 260 °C (500 °F) and then calcined at a temperature in the range of from 455 °C (851 °F) to 705 °C (1301 °F) for a period of 1 to 20 hours. This treatment effects conversion of the hydrogel to the corresponding crystalline gamma alumina.

As described in US 6187981 B1, the layer is applied by forming a slurry of the outer refractory oxide and then coating the inner core with the slurry by means well known in the art. Slurries of inorganic oxides can be prepared by means well known in the art which usually involve the use of a peptising agent. For example, any of the transitional aluminas can be mixed with water and an acid such as nitric acid, hydrochloric acid, or sulphuric acid to give a slurry. Alternatively, an aluminium sol can be made by, for example, dissolving aluminium metal in hydrochloric acid and then mixing the aluminium sol with the alumina powder.

The slurry may preferably contain an organic bonding agent to aid in the adhesion of the layer material to the inner core. Examples of this organic bonding agent include but are not limited to polyvinyl alcohol (PVA), hydroxy propyl cellulose, methyl cellulose, and carboxy methyl cellulose. The amount of organic bonding agent which may be added to the slurry may vary considerably, for example, from 0.1 to 3 wt. % of the slurry. How strongly the outer layer is bonded to the inner core can be measured by the amount of layer material lost during an attrition test, i.e., attrition loss. Loss of the second refractory oxide by attrition is measured by agitating the catalyst, collecting the fines and calculating an attrition loss. By using an organic bonding agent as described above, the attrition loss is typically less than 10 wt. % of the outer layer. Finally, the thickness of the outer layer may vary in the range of from 40 microns (0.00158 in) to 400 microns (0.0158 in), preferably in the range of from 40 microns (0.00158 in) to 300 microns (0.00181 in) and more preferably in the range of from 45 microns (0.00177 in) to 200 microns (0.00787 in). As used herein, the term "microns" means 10⁻⁶ metre.

Depending on the particle size of the outer refractory inorganic oxide, it may be necessary to mill the slurry in order to reduce the particle size and simultaneously give a narrower particle size distribution. This can be done by means known in the art such as ball milling for times in the range of from 30 minutes to 5 hours and preferably in the range of from 1.5 to 3 hours. It has been found in the art that using a slurry with a narrow particle size distribution improves the bonding of the outer layer to the inner core.

The slurry may also contain an inorganic bonding agent selected from an alumina bonding agent, a silica bonding agent, or mixtures thereof. Examples of silica bonding agents include silica sol and silica gel, while examples of alumina bonding agents include alumina sol, boehmite, and aluminium nitrate. The inorganic bonding agents are converted to alumina or silica in the finished catalyst composition. The amount of inorganic bonding agent typically varies in the range of from 2 to 15 wt. % as the oxide, and based on the weight of the slurry.

Coating of the inner core with the slurry can be accomplished by means such as rolling, dipping, spraying, etc. One preferred technique involves using a fixed fluidised bed of inner core particles and spraying the slurry into the bed to coat the particles evenly. The thickness of the layer may vary, but is typically in the range of from 40 microns (0.00158 in) to 400 microns (0.0158 in), preferably in the range of from 40 microns (0.00158 in) to 300 microns (0.0118 in) and most preferably in the range of from 45 microns (0.001 77 in) to 200 microns (0.00787 in). The optimum layer thickness depends on the use for the catalyst and the choice of the outer refractory oxide. Once the inner core has been coated with the layer of outer refractory inorganic oxide, the resultant layered support may be dried at a temperature in the range of from 100 °C (212 °F) to 320 °C (608 °F) for a time in the range of from 1 to 24 hours and then calcined at a temperature in the range of from 400 °C (752 °F) to 900 °C (1652 °F) for a time in the range of from 0.5 to 10 hours to effectively bond the outer layer to the inner core and provide a layered catalyst support. The drying and calcining steps may be combined into a single step.

When the inner core is composed of a refractory inorganic oxide (inner refractory oxide), it is necessary that the outer refractory inorganic oxide be different from the inner refractory oxide. Furthermore, it is also required that the inner refractory inorganic oxide has a substantially lower adsorptive capacity for catalytic metal precursors relative to the outer refractory inorganic oxide.

Having obtained the layered catalyst support as described in US 6187981 B1, catalytic metals as hereinbefore described can be dispersed on the layered support by means known in the art. Thus, a platinum group (Group VIII (IUPAC 8-10) of the Periodic Table of Elements) metal, a promoter metal, and a modifier metal can be dispersed on the outer layer.

These catalytic metal components can be deposited on the layered support by any manner known in the art, for example, by impregnating the layered support with a solution (preferably aqueous) of a decomposable compound of the metal or metals. By "decomposable" is meant that upon heating the metal compound is converted to the metal or metal oxide with the release of by-products. Examples of decomposable compounds of the platinum group (Group VIII (IUPAC 8-10) of the Periodic Table of Elements) metals are chloroplatinic acid, ammonium chloroplatinate, bromoplatinic acid, dinitrodiamino platinum, sodium tetranitroplatinate, rhodium trichoride, hexaamminerhodium chloride, rhodium carbonylchloride, sodium hexanitrorhodate, chloropalladic acid, palladium chloride, palladium nitrate, diamminepalladium hydroxide, tetraamminepalladium chloride, hexachloroiridate (IV) acid, hexachloroiridate (III) acid, ammonium hexachloroiridate (III), ammonium aquohexachloroiridate (IV), ruthenium tetrachloride, hexachlororuthenate, hexaammineruthenium chloride, osmium trichloride, and ammonium osmium chloride. Examples of decomposable promoter metal compounds are the halide salts of the promoter metals. A preferred promoter is tin and preferred decomposable compounds are stannous chloride or stannic chloride. Examples of decomposable modifier compounds of the alkali and alkaline earth metals are the halide, nitrate, carbonate or hydroxide compounds, e.g., potassium hydroxide, lithium nitrate.

All three types of metals can be impregnated using one common solution or they may be sequentially impregnated in any order. A preferred impregnation procedure involves the use of a steam-jacketed rotary dryer, wherein the support is immersed in the impregnating solution containing the desired metal compound contained in the dryer and the support is tumbled therein by the rotating motion of the dryer. Evaporation of the solution in contact with the tumbling support is expedited by applying steam to the dryer jacket. The resultant composite is allowed to dry under ambient temperature conditions, or dried at a temperature in the range of from 80 °C (176 °F) to 110 °C (230 °F), followed by calcination at a temperature in the range of from 200 °C (392 °F) to 700 °C (1292 °F) for a time in the range of from 1 to 4 hours, thereby converting the metal compound to the metal or metal oxide. For the platinum group (Group VIII (IUPAC 8-10) of the Periodic Table of Elements) metal compound, it is preferred to carry out the calcination at a temperature in the range of from 400 °C (752 °F) to 700 °C (1292 °F).

In one method of preparation, the promoter metal is first deposited onto the layered support described in US 6187981 B1 and calcined as described above and then the modifier metal and platinum group (Group VIII (IUPAC 8-10) of the Periodic Table of Elements) metal are simultaneously dispersed onto the layered support by using an aqueous solution which contains a compound of the modifier metal and a compound of the platinum group (Group VIII (IUPAC 8-10) of the Periodic Table of Elements) metal. The support is impregnated with the solution as described above and then calcined at a temperature in the range of from 400 °C (752 °F) to 700 °C (1292 °F) for a time in the range of from 1 to 4 hours.

An alternative method of preparation involves adding one or more of the metal components to the outer refractory oxide preparation mixture prior to applying it as a layer onto the inner core. For example, a decomposable salt of the promoter metal, e.g., tin (IV) chloride, can be added to a slurry composed of gamma alumina and aluminium sol. Further, either the modifier metal or the platinum group metal or both can be added to the slurry. Thus, in one method, all three catalytic metals are deposited onto the outer refractory oxide prior to depositing the second refractory oxide as a layer onto the inner core. The three types of catalytic metals can be deposited onto the outer refractory oxide powder in any order although not necessarily with equivalent results.

Another preferred method of preparation involves first impregnating the promoter metal onto the outer refractory inorganic oxide and calcining as described above. Next, a slurry is prepared (as described above) using the outer refractory inorganic oxide containing the promoter metal and applied to the inner core by means described above. Finally, the modifier metal and platinum group (Group VIII (IUPAC 8-10) of the Periodic Table of Elements) metal are simultaneously impregnated onto the layered composition which contains the promoter metal and calcined as described above to give the desired layered catalyst.

One particular method of preparation involves first preparing the outer refractory inorganic oxide using the oil drop method (as described above), except that the promoter metal is incorporated into the resulting mixture of hydrosol and the gelling agent prior to its being dropped into the oil bath. Thus, in this method, the aged and gelled spheres recovered from the oil bath contain the promoter metal. After washing, drying, and calcining (as described above), a slurry is prepared (as described above) using crushed spheres containing the promoter metal, and the slurry is applied to the inner core by means described above. The modifier metal and the platinum group (Group VIII (IUPAC 8-10) of the Periodic Table of Elements) metal are simultaneously impregnated onto the layered composition which contains the promoter metal and calcined (as described above) to give the desired layered catalyst. Another particular method of preparation involves preparing a slurry using the outer refractory inorganic oxide and then adding the promoter metal to the slurry. The slurry is then applied to the inner core by means described above. Finally, the modifier metal and the platinum group (Group VIII (IUPAC 8-10) of the Periodic Table of Elements) metal are simultaneously impregnated onto the layered composition and calcined to give the desired layered catalyst (as described above).

Other layered catalyst compositions and other methods of preparing such catalysts known in the art, for example as disclosed in US 4077912 A, US 4255253 A, and WO 98/14274 A1, may also be suitable for preparing dehydrogenation catalysts for use in the process of the present invention.

As a final step in the preparation of the afore-mentioned layered catalyst composition, the catalyst composition is reduced under hydrogen or other reducing atmosphere in order to ensure that the platinum group (Group VIII (IUPAC 8-10) of the Periodic Table of Elements) metal component is in the metallic state (zero valent). Reduction is carried out at a temperature of generally in the range of from 100 °C (212 °F) to 650 °C (1202 °F), preferably in the range of from 300 °C (572 °F) to 550 °C (1022 °F), for a time in the range of from 0.5 to 10 hours in a reducing environment, preferably dry hydrogen. The state of the promoter and modifier metals can be metallic (zero valent), metal oxide, or metal oxychloride.

The layered catalyst composition can also contain a halogen component which can be fluorine, chlorine, bromine, iodine, or mixtures thereof with chlorine and bromine preferred. This halogen component is present in an amount in the range of from 0.03 to 0.3 wt. %, with respect to the total weight of the catalyst composition. The halogen component can be applied by means well known in the art and can be done at any point during the preparation of the catalyst composition although not necessarily with equivalent results. It is preferred to add the halogen component after all the catalytic components have been added either before or after treatment with hydrogen.

Although it is preferred that all three metals are uniformly distributed throughout the outer layer of outer refractory oxide and substantially present only in the outer layer, the modifier metal can be present both in the outer layer and the inner core. This is because the modifier metal can migrate to the inner core, when the core is other than a metallic core.

The dehydrogenation conditions of step (i) of the process of the present invention may be conveniently selected to minimise cracking and formation of diene and aromatic by-products. When contacting the catalyst, the paraffins may be in the liquid phase or in a mixed vapour-liquid phase, but preferably the paraffins are in the vapour phase.

Typical dehydrogenation conditions include temperatures of preferably in the range of from 400 °C (752 °F) to 900 °C (1652 °F) and more preferably in the range of from 400 °C (752 °F) to 525 °C (977 °F), pressures of preferably in the range of from 1 kPa(g) (0.15 psi(g)) to 1013 kPa(g) (147 psi(g)), more preferably in the range of from 1 kPa(g) (0.15 psi(g)) to 345 kPa(g) (50 psi(g))and a preferred LHSV in the range of from 0.1 to 100 hr-1. As used herein, the abbreviation "LHSV" means liquid hourly space velocity, which is defined as the volumetric flow rate of liquid per hour divided by the catalyst volume, where the liquid volume and the catalyst volume are in the same volumetric units.

As per CA 870668 A, in one embodiment of the present invention, the LHSV is in the range of from 10 to 40 in combination with a superficial linear gas velocity (SLGV) of at least 0.305 metres per second, wherein the superficial linear gas velocity is a parameter well-known in the art for the study of the effect of the shape of the catalyst bed on the course of the reaction.

Generally for paraffins, the lower the molecular weight the higher the temperature required for comparable conversion. The pressure in the dehydrogenation zone is maintained as low as practicable, usually less than 345 kPa(g) (50 psi(g)), consistent with equipment limitations, to maximise chemical equilibrium advantages.

The feed stream may be admixed with a diluent material before, while, or after being flowed to the dehydrogenation zone. The diluent material may be preferably selected from hydrogen, steam, methane, ethane, carbon dioxide, nitrogen, and argon, and mixtures thereof. Hydrogen is the preferred diluent.

When hydrogen is utilised as the diluent it is preferably utilised in amounts sufficient to ensure a hydrogen to paraffin mole ratio in the range of from 0.1:1 to 40:1, more preferably in the range of from 1:1 to 10:1. The diluent hydrogen stream passed to the dehydrogenation zone will typically be recycled hydrogen separated from the effluent from the dehydrogenation zone in the hydrogen separation zone.

Water or a material which decomposes at dehydrogenation conditions to form water such as an alcohol, aldehyde, ether, or ketone, for example, may optionally be added to the dehydrogenation zone to minimise coke formation, either continuously or intermittently, in a preferred amount to provide, calculated on the basis of equivalent water, in the range of from 1 to 20,000 weight ppm of the feed stream. In the range of from 1 to 10,000 weight ppm of water addition typically gives best results when dehydrogenating paraffins having in the range of from 20 to 28 carbon atoms.

The monoolefin-containing dehydrogenated product stream from the paraffin dehydrogenation process of step (i) is typically a mixture of unreacted linear and branched paraffins, linear and branched olefins, in particular linear and branched monoolefins.

The dehydrogenated product stream may undergo a selective hydrogenation step to eliminate diolefins, as described in US 4523045 A prior to separation and recovery of the monoolefin products from the dehydrogenated product stream in step (ii) of the process of the present invention. It is also possible for the aromatic by-products and any remaining diolefins in the dehydrogenated product stream to be removed by a separate fixed bed adsorption step.

As hereinbefore described, surfactants for use in enhanced oil recovery applications are tailored depending upon the specific characteristics of the oil reservoir being treated. Consequently, branched monoolefins, linear monoolefins and/or mixtures thereof may be preferred for use in surfactant production as described herein, dependent upon the oil reservoir in which the surfactant is to be used. Similarly, alpha monoolefins, internal olefins and/or mixtures thereof may be preferred for use in surfactant production as described herein, dependent upon the oil reservoir in which the surfactant is to be used.

In step (ii) of the process of the present invention, the monoolefins and paraffins in the dehydrogenated product stream are separated in a separation zone by an adsorptive separation process. Thereafter, the paraffins may be recycled to the dehydrogenation zone and the monoolefins may be converted to surfactants.

As described in US 2008/0051619 A, an adsorptive separation process comprises an adsorption step wherein an adsorbent is brought into contact with the olefin-containing feed stream under adsorptive conditions and a desorption step wherein selectively adsorbed olefins are removed from the adsorbent under desorption conditions.

Adsorptive separations can be carried out in the vapour phase and in the liquid phase. However, liquid phase separations as preferred as lower temperatures can be used, thereby minimising olefin polymerisation.

The separation zone in step (ii) of the process of the present invention may comprise swing-bed type operations (for example, as disclosed in US 2920037 A) or continuous counter-current flow bed operations.

In a preferred embodiment, step (ii) of the process of the present invention is a simulated-moving-bed adsorptive separation process.

Typically, simulated-moving-bed processes simulate countercurrent movement of the adsorbent and the feed stream. Such processes are described in Kirk-Othmer Encyclopedia of Chemical Technology (4th Edition, Wiley, New York (1991), Volume 1, pp 583-585 (Adsorption, Liquid Separation section)).

A paper entitled "Olex: A Process for Producing High Purity Olefins" presented by J.A. Johnson, S. Raghuram and P.R. Pujado at the August 1987 Summer national meeting of the American Institute of Chemical Engineers describes a simulated-moving-bed (SMB) countercurrent adsorptive separation process for the separation of light straight-chain olefins from similar paraffins. Detailed descriptions of SMB are also given in US 3510423 A and US 2008/0051619 A1.

As indicated in US 2008/0051619 A, SMB processes often include at least three or four separate steps. Said steps are performed sequentially in separate zones within a mass of adsorbent retained in one or more vertical cylindrical adsorption chambers. The afore-mentioned zones are usually made up of a plurality of adsorbent beds.

The adsorbent beds are contained in one or more vertical vessels which may be collectively referred to as the "adsorbent chamber".

As described in US 2008/0051619 A1, the adsorbent beds are structurally separated from one another by a horizontal liquid collection/distribution grid, each grid being connected to a transfer line defining a transfer point at which process streams such as the feed stream and raffinate and extract streams enter or leave the vertical adsorption vessels.

In step (ii) of the process of the present invention, olefins from the dehydrogenation product stream of step (i) are selectively adsorbed by the adsorbent and the resulting "raffinate stream" comprises hydrocarbons, in particular paraffins from the feed stream, which are less selectively adsorbed by the adsorbent. The raffinate steam is then removed from the adsorption vessel.

Adsorbents that may be conveniently used in step (ii) of the process of the present invention may be varied depending upon the nature of the olefins to be extracted from the feed stream. Examples of adsorbents that may be used include molecular sieves.

Examples of preferred adsorbents are molecular sieves formed from inorganic oxides such as silica and alumina, that is to say, aluminosilicates. Aluminosilicates include the well known commercially available zeolites such as zeolite Y and zeolite X. Examples of adsorptive separation processes utilising crystalline aluminosilicate adsorbents are disclosed in US 3617504 A, US 3878128 A, US 3929669 A, US 3969223 A, US 4036744 A, US 4048111 A and US 4523045 A.

Molecular sieve zeolites contain exchangeable cationic sites. By ion-exchange using known methods, it is possible for said sites to contain one or more metal cations selected from the metals of Groups I-A, I-B, II-B and II-A (IUPAC 1, 11, 12 and 2) of the Periodic Table of Elements. Such ion exchange typically effects an increase in capacity of the zeolites for the adsorption of olefins. Preferred metal cations for ion exchange include beryllium, lithium, sodium, magnesium, potassium, calcium, rubidium, strontium, caesium, barium, gold, copper, silver, zinc and cadmium.

In one embodiment, it is preferred that said metal cations are selected from Groups I-A, I-B and II-A (IUPAC 1, 11 and 2) of the Periodic Table of Elements. It is particularly preferred that the metal cation is sodium.

However, in another embodiment, silver- and copper-exchanged zeolites, especially silver- and copper-exchanged Type X structured zeolites, are particularly preferred as adsorbents. Such zeolites are known in the art and are described, for example, in US 3718580 A, US 3755153 A and US 3755540 A.

Furthermore, the adsorbent for use in step (ii) of the process of the present invention may be a silicalite, that is to say, a very high silica to alumina ratio molecular sieve which is not a zeolite due to its lack of ion exchange capacity. Such materials are described in US 4061724 A, US 4073865 A and US 4104294 A.

ZSM type zeolites may also be used as the adsorbent in the process of the present invention. Such inorganic oxide molecular sieves are described in US 3702886 A (ZSM-5), US 3832449 A (ZSM-12), US 4016245 A (ZSM-35) and US 4046859 A (ZSM-38).

US 2008/0051619 A1 and US 6225518 B1 describe an example of a preferred adsorbent which is an attrition resistant particle of about 20-40 mesh (U.S.) size formed by extrusion or spray drying an admixture of a binder such as clay or alumina and a type X or type Y zeolite. The type X and type Y zeolites are described in US 2822244 A and US 3130007 A, respectively. Said zeolites may be ion exchanged to replace native sodium with one or more other cations selected from alkali metals and/or alkaline-earth metals. Preferred metals are one or more of lithium, potassium, calcium, strontium and barium. A particularly preferred adsorbent is a sodium form 13X zeolite.

The monoolefins extracted in step (ii) from the dehydrogenation product stream of step (i) by the adsorbent may be desorbed therefrom by using a desorbent, that is to say, a material which facilitates desorption of the monoolefins from the absorbent to form a so-called "extract stream".

Preferred desorbents for use in step (ii) of the process of the present invention are liquids which are able to displace monoolefins from the adsorbent with reasonable mass flow rates whilst not being so strongly adsorbed themselves as to unduly prevent olefins from displacing the desorbent from the adsorbent in a subsequent adsorption cycle.

Furthermore, preferred desorbents are compatible with the adsorbent and the feed stream. In particular, it is preferred that said desorbents should not reduce or destroy the capacity of the adsorbent or selectivity of the adsorbent for monoolefins vis-à-vis paraffins in the dehydrogenation product stream of step (i). In addition, desorbents should be inert materials, that is to say, said materials should not chemically react with or cause a chemical reaction with components of the feed stream, raffinate stream and extract stream.

Desorbents should also be easily separated from the extract and raffinate components, for example by fractionation.

Desorbents that may be conveniently used in the process of the present invention are compounds having in the range of from 5 to 18 carbons, in particular, hydrocarbons such as paraffins, naphthenic hydrocarbons, olefins and aromatic hydrocarbons having in the range of from 5 to 18 carbons.

The extract stream comprising monoolefins having in the range of from 20 to 28 carbon atoms and the raffinate stream generally are passed to separation means, typically fractional distillation columns, where at least a portion of desorbent is recovered and an extract product comprising olefins having in the range of from 20 to 28 carbon atoms and a raffinate product are produced.

One operational problem commonly described in the art in relation to the adsorptive separation of olefins is the possible accumulation of certain compounds present in the feed stream, on the active sites of the adsorbent. Such compounds, for example aromatic hydrocarbons and diolefins, act as poisons by possibly binding so tightly to the active sites of the adsorbent that the desorption procedure used for olefin recovery cannot not remove them.

Accordingly, the capacity of the adsorbent and thus the overall adsorptive separation process may decrease as such compounds accumulate on the adsorbent. Methods employed to prevent poisons from deactivating the molecular sieves used to separate olefins are described in US 5276246 A, US 5300715 A and US 6106702 A.

Hence, in a preferred embodiment of the process of the present invention, a guard bed, for example a molecular sieve guard bed may be used to remove aromatic hydrocarbon contaminants from the dehydrogenation product stream of step (i) in order to prevent such contaminants from deactivating the adsorbent bed used to separate olefins from paraffins in the dehydrogenation product stream in step (ii). The guard bed may be the same or different from the adsorbent bed used to effect separation of olefins from the feed stream. Suitable guard beds include those described in US 6102702 A and US 6225518 B1. A preferred molecular sieve for use in the guard bed is a lithium exchanged X zeolite.

US 5300715 A, US 6106702 A and US 6225518 B1 describe methods to regenerate molecular sieves used as a guard bed and/or as the adsorbent bed in order to remove remaining diolefins (for example, that were not removed in a previous optional selective hydrogenation step) and aromatic hydrocarbons therefrom and allow said molecular sieves to be re-used.

Adsorption and desorption conditions used in step (ii) of the process of the present invention may be varied depending upon on the composition of the adsorbent and the dehydrogenation product stream of step (i). However, the adsorptive separation process in step (ii) of the process of the present invention may be preferably carried out at a temperature in the range of from 20 to 250 °C, more preferably in the range of from 40 to 200 °C and most preferably in the range of from 50 to 150 °C. Step (ii) of the process of the present invention may be preferably carried out at a pressure sufficient to maintain the process fluids in liquid phase, for example at a pressure in the range of from atmospheric pressure to 4.5 MPa. Desorption conditions are generally similar to adsorption conditions, that is to say at a temperature in the range of from 20 to 250 °C, more preferably in the range of from 40 to 200 °C and most preferably in the range of from 50 to 150 °C and a pressure sufficient to maintain the process fluids in liquid phase, for example at a pressure in the range of from atmospheric pressure to 4.5 MPa.

The process of the present invention is not limited to any one particular flow scheme.

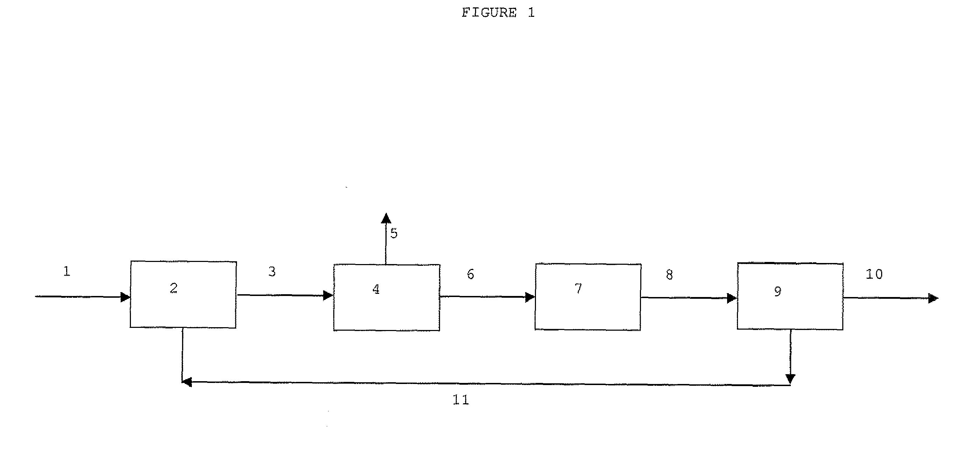

However, in a preferred embodiment of the present invention, the process may be configured substantially in the manner shown in the scheme of Figure 1. Briefly, a feed stream containing Fischer-Tropsch derived paraffins (1) combines with recycled hydrogen to form a dehydrogenation reactant stream that is heated and contacted with a dehydrogenation catalyst in a fixed bed in a dehydrogenation zone (2) maintained at dehydrogenation conditions. The effluent of the fixed catalyst bed, which is referred to herein as the dehydrogenation reactor effluent stream (3), is cooled, partially condensed, and passed to a vapour-liquid separator (4). The vapour-liquid separator produces a hydrogen-rich vapour phase (5) and a hydrocarbon-rich liquid phase (6). Diolefins in the hydrocarbon-rich liquid phase (6) may be optionally hydrogenated to monoolefins. The condensed liquid phase (6) recovered from the separator then passes to a stripping column (7), which removes all compounds which are more volatile than the lightest hydrocarbon which is desired. The monoolefin-containing net stream is referred to herein as the dehydrogenated product stream (8). The dehydrogenated product stream is passed to a separation zone as described herein (9) to separate the monoolefins (the so-called "extract stream") (10) from the unreacted paraffins (11) by an adsorptive separation process, which unreacted paraffins (the so-called "raffinate stream") (11) may, in turn, be optionally recycled to the dehydrogenation zone (2). In a preferred embodiment of the process of the present invention, said dehydrogenated product stream (8) is passed over a guard bed prior to entering the separation zone (9) in order to remove aromatic by-products and any remaining diolefins therefrom.

In a preferred embodiment of the process of the present invention, at least a portion of the separated monoolefins is contacted with a sulphonating agent and the resulting product is subsequently treated to form an olefin sulphonate surfactant, in particular an internal olefin sulphonate surfactant.

The sulphonation may be carried out batchwise, semi-continuously or continuously, preferably continuously.

Sulphonation and subsequent treatment to form an olefin surfactant sulphonate may be conveniently carried out in accordance with the methods described in US 4393937 A, EP 0351928 A1 and EP 0482687 A1.

Examples of sulphonating agents that may be conveniently used include not only sulphur trioxide (SO₃), but also any compounds or complexes which contain or yield SO₃. However, sulphur trioxide per se is particularly preferred for use as a sulphonating agent.

Preferably, the sulphonation reaction takes place between the one or more monoolefins and a sulphonating agent, for example in a film reactor, in a mol ratio of sulphonating agent to monoolefin in the range of from 2.0 to 0.9, more preferably in the range of from 1.3 to 1.0, while cooling the reactor with a cooling means having a temperature preferably not exceeding 35 °C and treating the reaction product to form the olefin sulphonate surfactant.

The reaction between the sulphonating agent and a monoolefin yields an intermediate believed to be in the nature of a sultone. The intermediate is subsequently treated to form an olefin sulphonate surfactant.

In one embodiment of the present invention, said treatment may comprise neutralisation by reaction with a base, preferably an alkali or alkaline earth metal hydroxide, oxide or carbonate, followed by a subsequent after-treatment step, i.e. a so-called hydrolysis step.

The neutralisation/hydrolysis may be preferably carried out continuously and at a temperature in the range of from 20 to 50 °C, more preferably in the range of from 30 to 45 °C. Hydrolysis is preferably carried out continuously and at a higher temperature than the neutralisation step, for example, up to and including 190 °C.

Another embodiment of the present invention, provides for the use of monoolefins prepared by the process of the present invention in the manufacture of olefin sulphonate surfactants, in particular internal olefins sulphonates.

The present invention further provides for the use of olefin sulphonate surfactants, and in particular internal olefin sulphonates, for enhanced oil recovery, wherein said olefin sulphonate surfactants have been made by the afore-mentioned sulphonation process, and wherein at least a portion of the monoolefins reacted in said sulphonation process are from the afore-mentioned extract stream.

The following Examples are provided to further illustrate certain specific aspects of the present invention and are not intended to limit the scope of the present invention in any way.

### EXAMPLES

### Example 1

### (i) Preparation of Dehydrogenation Catalyst

A paraffin dehydrogenation catalyst was prepared according to Example 1 (catalyst A) of US 4430517. The resulting catalyst included 0.8 wt. % platinum, 0.5 wt. % tin, 2.7 wt. % potassium and 1.3 wt. % chlorine on a gamma-alumina support. The atomic ratio of potassium to platinum for this catalyst was 16.8.

The dehydrogenation catalyst was prepared by dissolving substantially pure aluminium pellets in a hydrochloric acid solution. An amount of stannic chloride was added to the resulting solution to provide a final composite containing 0.5 wt. % tin and stirred to distribute the tin component evenly throughout the mixture. Hexamethylenetetramine was added to the resulting tin mixture and the resulting tin-amine mixture was dropped into an oil bath in a manner to form spherical particles having an average particle diameter of about 1/16 inch. The spheres were aged, washed with an ammoniacal solution, dried and calcined to form a spherical gamma-alumina carrier material. The resulting spheres contained about 0.5 wt. % tin in the form of tin oxide. More details about the method of preparing the alumina carrier material are disclosed in US 2620314 A.

The tin-alumina composite was contacted with a deionized solution of chloroplatinic acid and hydrochloric acid (2 wt. % based on alumina weight) in a rotary drier for 15 minutes at room temperature. The amount of chloroplatinic acid used was the amount necessary to incorporate 0.8 wt. % platinum into the tin-alumina composite. The solution was then heated and purged with nitrogen to remove water resulting in a platinum-chlorine-tin-alumina composite. The incorporated chlorine was removed by heating the platinum-chlorine-tin-alumina composite to 550 °C and treating the composite with a 50/50 air/80 °C steam mixture at a gas hourly space velocity (GHSV) of 300 hr⁻¹. After treatment with the air/steam mixture, the platinum-tin-alumina composite contained less than 0.1 wt. % chlorine.

The platinum-tin-alumina composite was contacted with a deionized water solution of potassium nitrate. The amount of potassium nitrate used was the amount necessary to incorporate 2.7 wt. % of potassium in the platinum-tin-alumina composite. The water was removed from the platinum-tin-potassium-alumina composite by heating the composite to 100 °C under a purge of dry air (1000 hr⁻¹GHSV) for 0.5 hour. The temperature was raised to 525 °C and the platinum-tin-potassium alumina composite was treated with a stream of hydrochloric acid (12 cc/hr, 0.9 M HCl) and a stream of 50/50 air/80 °C steam mixture (300 hr⁻¹ GHSV) to incorporate chlorine into the platinum-tin-potassium-alumina composite. The platinum-tin-potassium-chlorine-alumina composite was dried at 525 °C under a purge of dry air (1000 hr⁻¹ GHSV). The resulting catalyst spheres had an average particle diameter of 1/16 inch and were crushed and sized into 6-20 mesh particle before testing.

Stainless steel tubing, 1 inch OD, 0.6 inch ID and 26 inches long, was used as a de-hydrogenation reactor. A thermowell extended 20 inches from the top of the stainless steel reactor tube. To load the reactor tube, the reactor tube was inverted and a piece of glass wool was transferred down the wall of the reactor tube, over the thermowell and positioned at the bottom of the reactor tube to serve as a plug for the reactor tube. Silicon carbide (20 mesh) was added to a depth of about 6 inches to the reactor tube. A second piece of glass wool was placed over the silicon carbide. A mixture of 6.0 grams of platinum-tin on alumina catalyst particles (6-20 mesh) and 45 grams of fresh silicon carbide (60-80 mesh) was added to the reactor tube in two parts. The two-part addition distributed the catalyst evenly in the reactor tube and resulted in a catalyst bed of about 10 inches in length. A third piece of glass wool was added to the top of the catalyst in the reactor tube. Silicon carbide (20 mesh) was layered onto the third piece of glass wool. A fourth piece of glass wool was positioned over the silicon carbide to serve as a plug for the bottom of the reactor tube. To monitor the temperature of the reaction at various points in the reactor tube, a multipoint thermocouple was inserted into the thermowell of the reactor tube. The temperature above, below and at three different places in the catalyst bed was monitored. The reactor tube was inverted and installed in the furnace. The reactor tube was purged with nitrogen. The reactor tube was heated to the operating temperature of 425 °C over a four-hour period under flowing nitrogen (250 standard litres per hour). Once the temperature of 425°C was obtained, the reactor tube was held at the operating temperature for an additional two hours. The catalyst was presulphided by flowing a 1 % mixture of hydrogen sulfide gas in hydrogen gas at 425 °C for five minutes through the reactor tube. After 5 minutes, the hydrogen sulfide in hydrogen gas flow was switched to a hydrogen gas flow through the reactor tube.

After presulphiding the catalyst, the reactor tube was maintained at 425 °C for eight hours. After eight hours, the reactor tube pressure was increase to 25 psig with hydrogen gas. The paraffin feedstock was pumped through the reactor tube at a flow rate of 40 g/hr at a hydrogen flow rate of 125 standard litres per hour. After four hours, the paraffin feedstock stream was increased to 80 g/hr. After obtaining a flow rate of 80 g/hr, the reactor tube temperature was raised to 460 °C. The reactor tube was sampled every eight hours after obtaining the operating temperature of 460 °C.

### (ii) Dehydrogenation of C20-C24 paraffin carbon fraction derived from Fischer Tropsch process streams (prophetic)

Two feedstocks differing in composition and branched content are processed in the laboratory to olefins by dehydrogenation.

The two feedstocks are obtained from different Fischer-Tropsch derived process streams.

The first C20-C24 paraffinic feedstock is obtained from the Heavy Paraffin Synthesis (HPS) reactor stream by distillation followed by hydrogenation and consists of a mixture of linear paraffins and branched paraffins in the ratio 94 wt. %/6 wt. %.

The second C20-C24 paraffinic feedstock is obtained by distillation of a selected process stream down stream of the Heavy Paraffin Cracking (HPC) reactor after the Catalytic Dewaxing Unit. The C20-C24 feedstock contains more than 99 wt. % branched paraffins.

The composition of the two feedstocks is given in Table 1.

**Table 1**

| Compositional analyses of C20-C24 carbon range paraffinic feedstocks | | |
|---|---|---|
| | Ex. Heavy Paraffin Syntheses (HPS) Unit (wt. %) | Ex. Heavy Paraffin Cracking (HPC) Unit (wt. %) |
| < C20 | 1.17 | 2.02 |
| C20 | 9.44 | 20.78 |
| C21 | 23.33 | 26.59 |
| C22 | 23.82 | 25.81 |
| C23 | 21.24 | 17.20 |
| C24 | 15.62 | 7.20 |
| > C24 | 5.38 | 0.40 |
| Branched paraffin content | 6 | > 99 |

The dehydrogenation unit is operated such that the conversion of the paraffin feedstock is below 15%. The resulting product is collected and analysed for olefin content by Gas chromatographic (GC) analyses and Bromine analyses. ¹H NMR and ¹³C NMR are used to assess the amount of branched products.

### (iii) Separation of the C20-24 Dehydrogenation Products (prophetic)

The dehydrogenation product mixture of step (ii) is separated in a separation unit according to methods described in the art.

The adsorbent is selected in accordance with the general methodologies described in paragraphs [0030]-[0031] of US 2008/0051619 A1 and columns 5 and 6 of US 3755540 A.

## Claims

1. A process for the preparation and recovery of olefins, said process comprising:-
(i) passing a feed stream comprising paraffins having in the range of from 20 to 28 carbon atoms to a dehydrogenation zone, operating the dehydrogenation zone at dehydrogenation conditions sufficient to dehydrogenate paraffins, and recovering from the dehydrogenation zone a dehydrogenation product stream comprising monoolefins and paraffins wherein the monoolefins in the dehydrogenated product stream have in the range of from 20 to 28 carbon atoms; and
(ii) passing the dehydrogenation product stream to a separation zone and contacting said dehydrogenation product stream with a bed of adsorbent under conditions which cause the selective retention of the monoolefins having in the range of from 20 to 28 carbon atoms on the adsorbent and recovering the retained monoolefins having in the range of from 20 to 28 carbon atoms from the adsorbent by contacting the adsorbent with a desorbent.

2. Process according to Claim 1, wherein the feed stream is derived from a Fischer-Tropsch process.

3. Process according to Claim 1 or 2, wherein said monoolefins are branched olefins having in the range of from 20 to 28 carbon atoms.

4. Process according to any one of Claims 1 to 3, wherein the dehydrogenation zone comprises a dehydrogenation catalyst comprising at least one platinum group (Group VIII (IUPAC 8-10) of the Periodic Table of Elements) metal, a promoter metal, a modifier metal, and a porous carrier material.

5. Process according to any of Claims 1 to 4, wherein the adsorbent comprises a molecular sieve.

6. Process according to any one of Claims 1 to 5, wherein the desorbent is selected from paraffins, naphthenic hydrocarbons, olefins and aromatic hydrocarbons having in the range of from 5 to 18 carbons.

7. Process according to any one of Claims 1 to 6, wherein step (ii) comprises a simulated-moving-bed adsorptive separation process.

8. Process according to any one of Claims 1 to 7, wherein at least a portion of the monoolefins having in the range of from 20 to 28 carbon atoms separated in step (ii) is contacted with a sulphonating agent and the resulting product is subsequently treated to form one or more olefin sulphonate surfactants.

9. Use of monoolefins having in the range of from 20 to 28 carbon atoms prepared by the process of any one of Claims 1 to 7 in the manufacture of olefin sulphonate surfactants.

10. Use of olefin sulphonate surfactants made by the process of Claim 8 for enhanced oil recovery.
